# EUROPEAN PATENT SPECIFICATION

(11) **EP 2 588 437 B1**
(45) Date of publication and mention of the grant of the patent: **02.01.2019**
(21) Application number: 11729803.4
(22) Date of filing: 15.06.2011
(51) Int. Cl.: C07C 43/13, C11D 1/722, C08G 65/26

(54) **BRANCHED SECONDARY ALCOHOL ALKOXYLATE SURFACTANTS AND PROCESS TO MAKE THEM**
VERZWEIGTE SEKUNDÄRE ALKOHOLALKOXYLATTENSIDE UND HERSTELLUNGSVERFAHREN DAFÜR
TENSIOACTIFS DE TYPE ALKOXYLATE D'ALCOOLS SECONDAIRES RAMIFIÉS ET LEUR PROCÉDÉ DE FABRICATION

(30) Priority: 29.06.2010 US 359437 P
(43) Date of publication of application: 08.05.2013
(73) Proprietor: Dow Global Technologies LLC, Midland, MI 48674 (US)
(72) Inventor: YU, Wanglin, Pearland, TX 77584 (US); MAYNARD, Shawn, J., Angleton, TX 77515 (US); AGUILAR, Daniel, A., Lake Jackson, TX 77566 (US)
(74) Representative: Beck Greener
(86) International application number: PCT/US2011/040426
(87) International publication number: WO 2012/005897

(56) References cited:
- WO-A1-2005/085321
- WO-A1-2009/000852
- DE-A1- 10 017 197
- US-A- 2 617 830
- US-A1- 2010 075 389

## Description

### Field of the Invention

The invention relates to alkoxylate compositions and to processes for making and using them. The alkoxylate compositions exhibit a narrow molecular weight distribution and contain low levels of residual alcohol.

### Background of the Invention

Alcohol ethoxylates are an industrially important class of materials that find use in a wide variety of applications, for instance, as surfactants and detergents. Primary alcohol ethoxylates are conventionally prepared by base catalyzed ethoxylation of a primary alcohol. The simplicity of the manufacturing process and its ability to provide quality products (i.e., narrow molecular weight distribution and containing low levels of residual alcohol) allows a wide variety of these types of materials to be prepared.

For example, US 2010/075389 provides foam control agents and their use for controlling foam in fermentation processes. The foam control agents are of the formula R²R¹HCO-(CH₂CH₂O)*ₙ*-(CHR³CHR³O)*_{y}*-H. DE 10017197 relates to alcohol alkoxylates of general formula (I) R¹-O-(CH₂-CHR⁵-O-)*ᵣ*(CH₂-CH₂-O-)*ₙ*(CH₂)-CHR⁶-O-)*ₛ*(CH₂-CHR²-O-)*ₘ*H, wherein R¹ represents at least a mono-branched C₄₋₂₂ alkyl, R² represents C₃₋₄ alkyl, R⁵ represents C₁₋₄ alkyl, R⁶ represents methyl or ethyl, *n* represents a mean value of between 1 and 50, *m* represents a mean value of between 0 and 20, *r* represents a mean value of between 0 and 50, *s* represents a mean value of between 0 and 50, whereby *m* is at least 0.5, if R⁵ is methyl or ethyl, or if *r* has the value 0; or to alcohol alkoxylates of general formula (II) R³-O-(CH₂-CH₂-O-)*ₚ*(CH₂-CHR⁴-O-)*_{q}*H, wherein R³ represents a branched or unbranched C₄₋₂₂ alkyl, R⁴ represents C₃₋₄ alkyl, *p* represents a mean value between 1 and 50 and q represents a mean value of between 0.5 and 20. Said alcohol alkoxylates are used as low-foam, or foam-inhibiting surfactants. WO 2005/085321 relates to low foaming nonionic surfactants being alcohol alkoxylates comprising at least two different oxyalkylene - groups derivable from a mixture of linear an branched alcohols and their use. US 2617830 relates to polyglycol ethers of 7-ethyl-2-methyl-undecanol-4, having high surface-activity in aqueous solutions, and processes for their preparation. WO 2009/000852 relates to a process for the preparation of an alkoxylate composition, said process comprising the steps of: (a) introducing into a reactor system one or more compounds with one or more active hydrogen atoms, selected from the group comprising alkanoic acids, alkanoic amides, alkanoic ethanolamides, alcohols and alkylmercaptans, and a double metal cyanide catalyst; (b) contacting the one or more compounds with one or more active hydrogen atoms and the double metal cyanide catalyst with propylene oxide and/or butylene oxide to form a first product mixture, comprising compounds formed by the addition of one of more propylene oxide and/or butylene oxide units to the compound with one or more active hydrogen atoms, and DMC catalyst; (c) contacting the first product mixture with ethylene oxide to form a second product mixture comprising compounds formed by the addition of one of more ethylene oxide units to the compounds formed in step (b), and DMC catalyst. Also, an alkoxylate composition which comprises an alcohol having been reacted with one or more molar equivalents of PO and then one or more molar equivalents of EO.

In contrast to primary alcohols, highly branched secondary alcohols are considerably less reactive and therefore much more difficult to ethoxylate by the base catalyzed process. As a result, alternative procedures for manufacture of highly branched secondary alcohol ethoxylates have been developed.

A commonly used alternative is based on a two-step process. In step one, an alcohol or alcohol mixture is treated with ethylene oxide (EO) in the presence of a Lewis acid catalyst, BF₃ is commonly used, to add a small amount of EO to the alcohol. The low EO adduct is purified by thorough washing to remove the catalyst and by-products and then subjected to distillation to separate the desired product from unreacted alcohols and lower adducts. The purified low EO product (average 2-4 mole EO) is carried to step two in which a base-catalyzed conventional alkoxylation is performed to produce the final surfactant products.

The two-step process has a number of disadvantages. For instance, the product from the first step generally contains considerable amount of byproduct 1,4-dioxane that needs to be removed. In addition, the ethoxylate products typically exhibit an unfavorably broad molecular weight distribution and a large amount of unreacted alcohol starting material. As a result, if final materials of acceptable quality are to be prepared, isolation and purification of intermediates is needed. Such isolation and purification, and the additional second alkoxylation process, however, significantly increase the cost of the process and result in the generation of large amounts of waste.

New highly branched secondary alcohol alkoxylates that exhibit narrow molecular weight distributions and low content of residual alcohols, as well as low-cost and low waste-generating processes for making them, would be a significant advance in the art.

### BRIEF SUMMARY OF THE INVENTION

The invention relates to a composition comprising one or more alkoxylates of formula II: 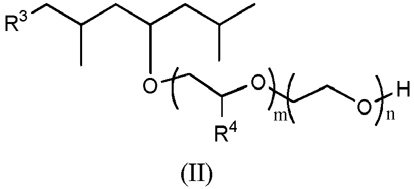 wherein R³ is H or iso-propyl and R⁴ is CH₃ or CH₂CH_{3;} m is 1-6; n is 1-40; wherein the polydispersity index of the alkoxylates is 1.15 or less, and wherein the composition comprises no more than 2 percent by weight of residual alcohol.

Above invention is embedded within a more general disclosure as described in the following.

In one aspect, the disclosure provides an alkoxylate composition that exhibits narrow molecular weight distribution and also contains low levels of residual unreacted alcohol. The composition comprises one or more alkoxylates of formula I: 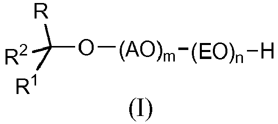 wherein AO, EO, m, n, R, R¹ and R² are as defined below.

In another aspect, the disclosure provides a process for making an alkoxylate of formula I. The process comprises: reacting under alkoxylation conditions a secondary alcohol having 7 to 16 carbon atoms and a branching degree of 3 or more with an alkylene oxide compound containing 3 or more carbon atoms followed by ethylene oxide. The alkoxylation is conducted in the presence of a double metal cyanide catalyst.

### DETAILED DESCRIPTION OF THE INVENTION

The disclosure, within which the invention as defined above is embedded, in a first aspect provides a composition comprising one or more alkoxylates of the formula I: 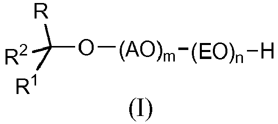 wherein AO is an alkyleneoxy containing at least 3 carbon atoms; EO is ethyleneoxy; m is 1-6; n is 1-40; R and R¹ are independently C₁-C₁₄ alkyl; and R² is H or C₁-C₁₃ alkyl, wherein the group formed by R, R¹, R² and the carbon to which they are attached contains 7 to 16 carbon atoms and has a branching degree of at least 3, provided that when R¹ is CH₃(CH₂)₂CH(C₂H₅)(CH₂)₂CH(CH₃)- and R² is H, then R is not CH₃.

Alkoxylates of formula I prepared according to the processes described herein have been surprisingly discovered to exhibit a narrow molecular weight distribution, represented by the materials' polydispersity index (weight average molecular weight/number average molecular weight (Mw/Mn) as determined by gel permeation chromatography). A narrow molecular weight distribution generally results in better surfactant performance. In some embodiments, the polydispersity index (PDI) of the alkoxylates is 1.15 or less, alternatively 1.1 or less.

In addition to exhibiting low PDI, alkoxylates of formula I prepared as described herein also contain surprisingly low levels of residual unreacted alcohols. The advantages of having low levels of alcohols include enhanced surface activity, low odor, and improved clarity of aqueous formulations. In some embodiments, the compositions contain 3 weight percent or less, alternatively 2 weight percent or less, alternatively 1 weight percent or less, or alternatively 0.5 weight percent or less of residual alcohols. Notably, the low residual alcohol content of the invention may be achieved without the need to use large amounts of ethylene oxide (EO). Although it is known that increasing the EO level in conventional surfactants may result in greater consumption of starting alcohol, the increased EO may also cause difficulty in achieving the desired cloud point for the surfactant.

In the alkoxylates of formula I, AO represents an alkyleneoxy group containing at least 3 carbon atoms. In some embodiments, AO is butyleneoxy (BO). In some embodiments, AO is propyleneoxy (PO).

Formula I includes variables "m" and "n" that describe the molar ratio of charged reagents. The reaction product produced between the reaction of the alcohol and the alkyleneoxy containing at least 3 carbon atoms, or the adduct thereof and the ethylene oxide is a distribution of oligomers that have on average the molar ratio of the charged reagents. Individually, "m" and "n" represent molar ratios of, respectively, alkoxylation with an alkyleneoxy containing at least 3 carbon atoms and ethoxylation. In some embodiments of the invention m is at least 1, alternatively at least 2. In some embodiments, m is 5 or less, alternatively 4 or less, or alternatively, 3 or less. In some embodiments, m falls in the range of 1 to 5, alternatively 2 to 5.

In some embodiments, n is at least 2, alternatively at least 3, alternatively at least 4, or alternatively at least 5. In some embodiments, n is 30 or less, alternatively 20 or less, alternatively 10 or less, or alternatively 9 or less. In some embodiments, n falls in the range 2 to 10, alternatively 3 to 9, or alternatively 5 to 9.

In the formula I alkoxylates, R, R¹, R² and the carbon to which they are attached form a group that is the organic residue of the highly branched secondary alcohol used to make the alkoxylate. In general, the group contains between 7 and 16 carbon atoms. In some embodiments, the group contains between 9 and 12 carbon atoms. The group also has a branching degree of 3 or more. In some embodiments of the invention, the branching degree is 4 or more. The term "branching degree" as used herein means the total number of methyl (-CH₃) groups minus 1. For instance, if there are four methyl groups, then the branching degree is 3. Compounds in which simultaneously R¹ is CH₃(CH₂)₂CH(C₂H₅)(CH₂)₂CH(CH₃)-, R² is H, and R is CH₃ are excluded as alkoxylates of the invention (i.e., compounds prepared from (3-methyl-6-ethyl)-2-nonanol as the secondary alcohol).

In some embodiments of the disclosure, R is C₃-C₁₂ alkyl, alternatively C₃-C₈ alkyl, or alternatively C₄-C₆ alkyl. In some embodiments, R contains at least 2 methyl groups.

In some embodiments of the disclosure, R¹ is C₃-C₁₂ alkyl, alternatively C₄-C₁₀ alkyl, or alternatively C₆-C₈ alkyl. In some embodiments, R¹ contains at least 2 methyl groups.

In some embodiments of the disclosure, R² is C₁-C₃ alkyl. In some embodiments, R² is H.

In embodiments of the invention, the alkoxylate is of the formula II: 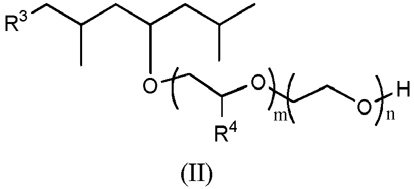 wherein R³ is H or iso-propyl and R⁴ is CH₃ or CH₂CH₃, m is 1-6, and n is 1-40, the polydispersity index of the alkoxylates is 1.15 or less, and the compositions contain 2 weight percent or less, as defined above.

In some embodiments of the invention, the alkoxylate is of the formula: 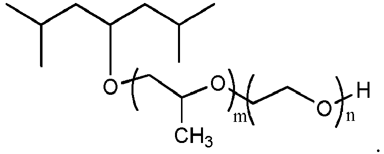 wherein m and n are as defined above.

In some embodiments, the alkoxylate is of the formula: 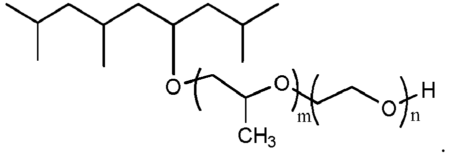 wherein m and n are as defined above.

In another aspect, the disclosure provides a process for making the alkoxylates of formula I. According to the process, a highly branched secondary alcohol is reacted with an alkylene oxide compound containing 3 or more carbon atoms, followed by ethylene oxide, under alkoxylation conditions. The catalyst used for the alkoxylations is a double metal cyanide compound.

The highly branched secondary alcohol is a compound containing 7 to 16 carbon atoms, a branching degree of 3 or more, and one hydroxy group. In some embodiments, the compound contains between 9 and 12 carbon atoms. In some embodiments, the branching degree is 4 or more.

In another aspect, the invention provides a process for making the alkoxylates of formula II. According to the process, a highly branched secondary alcohol is reacted with an alkylene oxide compound containing 3 or more carbon atoms, followed by ethylene oxide, under alkoxylation conditions. The catalyst used for the alkoxylations is a double metal cyanide compound. The highly branched secondary alcohol is a compound containing 9 to 12 carbon atoms, a branching degree of 3 or more, and one hydroxy group, selected from 2,6,8-trimethyl-4-nonanol and 2,6-dimethyl heptan-4-ol. Excluded from the process of the invention is the alcohol (3-methyl-6-ethyl)-2-nonanol. The alkylene oxide compound is propylene oxide or butylene oxide.

Prior to the alkoxylation reaction, it may be advantageous to dry the starting alcohol in order to reduce its water content. Known techniques may be used, including for instance application of reduced pressure, elevated temperature, nitrogen purge, or a combination of these. The water content may be reduced to, for example, 300 ppm or less, alternatively 200 ppm or less, or alternatively 100 ppm or less, or alternatively 50 ppm or less, or alternatively 25 ppm or less.

The alkylene oxide compound containing 3 or more carbon atoms is reacted with the alcohol under alkoxylation conditions. In a non-limiting embodiment illustrative of suitable alkoxylation conditions, this reaction may be carried out at an elevated temperature or temperatures ranging from about 80 °C to about 180 °C. In other non-limiting embodiments, the temperature may range from about 100°C to about 160°C. Pressures from about 14 psia to about 60 psia may, in certain non-limiting embodiments, be particularly efficacious, but other pressures may also be effectively employed. Those skilled in the art will be able to determine appropriate conditions with, at most, routine experimentation.

Following the alkoxylation with the alkylene oxide compound containing 3 or more carbon atoms, the product is then ethoxylated with ethylene oxide. As with the initial alkoxylation, this reaction may also be carried out at an elevated temperature or temperatures ranging from about 80 °C to about 180 °C or, for instance, from about 100°C to about 160°C. Pressures from about 14 psia to about 60 psia may, in certain non-limiting embodiments, be particularly efficacious, but other pressures may also be effectively employed.

The alkoxylation and ethoxylation reactions are conducted in the presence of an effective amount of a double metal cyanide compound as catalyst. The amount of the catalyst may, in some embodiments, range from about 0.0001 percent to about 0.1 percent by weight, based on the total charge of alcohol and oxides. Suitable double metal cyanide catalysts include those described in U.S. Patent 6,429,342, which is incorporated herein by reference. By way of example, Zn₃[Co(CN)₆]₂ may be used as the catalyst.

Following the alkoxylation reactions, the product may be discharged from the reactor directly to be packaged without removal of the catalyst. If desired, the product may be filtered prior to packaging or use, or treated by different means to remove or recover the catalyst, such as taught in US4,355,188; 4,721,818; 4,877,906; 5,010,047; 5,099,075; 5,416,241, each of which is incorporated herein by reference
The final formula I alkoxylate of the disclosure and the final formula II of the invention may be used in formulations and compositions in any desired amount. By way of example, when used as a surfactant, typical amounts in many conventional applications may range from about 0.05 to about 90 weight percent, more frequently from about 0.1 to about 30 weight percent, and in some uses from about 0.5 to about 20 weight percent, based on the total formulation. Those skilled in the art will be able to determine usage amounts via a combination of general knowledge of the applicable field as well as routine experimentation where needed.

Applications of the alkoxylates of the invention may include a wide variety of formulations and products. These include, but are not limited to, as surfactant, or wetting, dispersing, demulsifying, cleaning, foam controlling agents, or adjuvant, or combination of these functions in cleaners, detergents, hard surface cleaning formulations, polyurethanes, epoxies, emulsion polymerization, thermoplastics, metal products, agricultural products including herbicides and pesticides, oilfield products and processes, pulp and paper products, textiles, water treatment products, flooring products, inks, colorants, pharmaceuticals, cleaning products, personal care products, and lubricants. As an example of the dispersing application, the alkoxylates of the invention may be used as dispersing agents for fluororesins.

The following examples are illustrative of the invention but are not intended to limit its scope. Unless otherwise indicated, the ratios, percentages, parts, and the like used herein are by weight.

### EXAMPLES

### Raw Materials

2,6,8-Trimethylnonan-4-ol (TMN) and 2,6-dimethyl heptan-4-ol (diisobutyl carbinol or DIBC) are supplied by The Dow Chemical Company.

Double metal cyanide (DMC) catalyst is supplied by Bayer.

Ethylene Oxide (EO), propylene oxide (PO), and butylene oxide (BO) are supplied by The Dow Chemical Company.

### Manufacturing Equipment

DMC catalyzed surfactant samples are prepared using a semi-batch process in a 9 liter, stirred, baffled, jacketed reactor.

### Property Test Methods

Conventional GPC is used for general molecular weight analysis. Reported results are relative to linear polyethylene glycol standards, shown below. Polymer Laboratories PEG-10 Polyethylene glycol standards are used with 3^{rd} order fitting. Molecular weight (Mₙ, M_{w}, M_{z}) is measured with an Agilent 1100 system equipped with a Polymer Labs Mixed E column coupled to a Differential Refractive Index (RI) detector operated at 40°C. The chromatographic mobile phase is tetrahydrofuran (THF). Each sample (100 ul, 25.00 mg/mL) is dissolved in THF, injected twice, and eluted at 1.0 mL/min.

The amount of unreacted alcohol in alkoxylate samples is determined by gas chromatography. External alcohol standards dissolved in methanol are used. The standard stock solution (0.016 - 11.249 % (w/w)) is prepared by weighing the alcohol (0.06 g - 1.01 g) and methanol (10 g) into a glass vial. Low concentrations are created by dilution of existing samples (0.64 % (w/w)). Alkoxylate samples are prepared by dilution in methanol by mass to achieve the desired concentration. The alcohol concentration data reported are from single injections.

Surface tension is tested on a Krüss K100 Tensiometer using Wilhelmy plate method in de-ionized water at 25°C.

Skein wetting, or Draves Wetting, property is tested following ASTM Method D2281 at 25°C

Foaming properties are tested following the procedure of ASTM D1173 at 25°C.

### Example 1

### TMN/PO/EO Alkoxylate Surfactants

The alkoxylate is prepared by first forming the PO-TMN adduct by reaction between PO and TMN in the presence of the DMC catalyst followed by reaction between EO and the PO-alcohol adduct. TMN alcohol is stripped at 80°C under vacuum with nitrogen sweep until the water content reaches less than 200 ppm (24 ppm). DMC catalyst (0.126 g) is then slurried in 1250 g of the dried starter alcohol (TMN). The TMN Alcohol / DMC catalyst slurry is charged to a 9-liter alkoxylation reactor and purged with nitrogen. The reactor is sealed and pressured with nitrogen to 16-20 psia, then heated with agitation to reaction temperature (130°C). The DMC catalyst is activated with 210 g of PO at 130°C, and then 555 g PO (765 g total) are added continuously (5 g/min) with stirring followed by a 2 hr digest period (130°C) to consume residual oxide. A sample (100 g) is removed from the reactor and measured for hydroxyl analysis (5.546% hydroxyl or 307 molecular weight corresponding to the 2 mole propoxylate). To the remaining PO-alcohol adduct 1110 g of EO is added at (5 g/min) with stirring at 130°C followed by a 2 hr digestion period (130°C) with intermediate sampling (200 g). This is followed by a second ethylene oxide (775 g) feed, digest, and intermediate sampling (221 g). A third ethylene oxide (450 g) feed and digest affords the alkoxylate product, TMN/2PO/9EO (the hydroxyl content is2.420% or 702 molecular weight by hydroxyl analysis, corresponding to the foregoing formula). As listed in Table 1, the TMN/2PO/9EO sample contains 1.3 wt% of unreacted alcohol residue and has PDI at 1.07.

Following the same procedure, other TMN/PO/EO products are prepared as listed in Table 1. All the samples contain less than 2 wt% unreacted alcohol residue and show PDI of less than 1.15.

Physical properties of the samples are tested and reported in Table 1. All the samples show good surface tension reduction, reducing the surface tension of water. The wetting properties, reflected by the Draves Wetting results and contact angles on Teflon film are also favorable.

**Table 1. Property Results for TMN Ethoxylates and Alkoxylates**

| Description | Unreacted TMN (wt%) | Surface Tension 1wt% dynes cm | Contact Angle (deg)² | Draves Wetting (wt% for 20 sec. wetting) | Mw | PDI | Ross Miles Foam (0.1 wt% solution) | | |
|---|---|---|---|---|---|---|---|---|---|
| | | | | | | | Initial (mm) | 5 Min (mm) | Rate (mm/ min) |
| TMN/8EO¹ (Anhydrous) | 4.7 | 25.9 | 39.9 | 0.05 | 726 | 1.17 | 35 | 0 | -7 |
| TMN/2PO/ 7EO | 1.9 | 26.9 | 46.7 | 0.05 | 746 | 1.09 | 100 | 55 | -6 |
| TMN/2PO/ 9EO | 1.3 | 26.7 | 43.9 | 0.05 | 805 | 1.07 | 120 | 70 | -10 |
| TMN/5PO/ 5EO | 0.4 | 27.3 | 60.7 | 0.09 | 785 | 1.06 | 20 | 8 | -2 |
| TMN/5PO/ 7EO | 0.2 | 27.5 | 58.3 | 0.07 | 857 | 1.05 | 75 | 30 | -8 |
| TMN/5PO/ 9EO | 0.1 | 28.6 | 54.4 | 0.07 | 930 | 1.04 | 110 | 50 | -12 |

| | | | | | | | | | |
|---|---|---|---|---|---|---|---|---|---|
| ¹Comparative example ²Measurements made using 5 µL drops (0.1wt% solution in de-ionized water) on Teflon tape at 20°C. | | | | | | | | | |

### Comparative Example 1

### Direct Ethoxylation of TMN alcohol catalyzed by DMC

DMC catalyst (0.1037 g) is dispersed in 772.4 g of starter alcohol (TMN) that has been dehydrated (90°C, under vacuum, with nitrogen sweep, until water is less than 200 ppm (25 ppm)). The TMN/DMC mixture is charged to a 9-liter reactor and purged with nitrogen. The reactor is sealed and pressured with nitrogen to 16-20 psia, then heated with agitation to reaction temperature (130°C). The DMC catalyst is activated (125 g EO, 130°C, 20 psia nitrogen), and then 320 g EO is added (445 g total) continuously (5 g/min) with stirring resulting in the ethoxylate product after 70 min digestion period (130°C). An intermediate sample (101 g) is removed followed by a second ethylene oxide (935 g) feed at 5 g / min and digest period. The reaction product measures 3.271 % hydroxyl or 520 molecular weight, corresponding to the 8 mole ethoxylate. The resulting product contains 4.7 wt% unreacted alcohol residue and has a PDI of 1.17.

### Example 2

### DIBC/PO/EO Alkoxylate Surfactants

The alkoxylate is prepared by first forming the PO-DIBC adduct by reaction between PO and DIBC in the presence of the DMC catalyst followed by reaction between EO and the PO-alcohol adduct. DIBC is stripped at 90°C under vacuum with nitrogen sweep until water content is less than 200 ppm (27 ppm). DMC catalyst (0.24 g) is slurried in 621 g of the dehydrated starter alcohol (DIBC). The DIBC / DMC catalyst slurry is charged to a 9 liter alkoxylation reactor and purged with nitrogen. The reactor is sealed and pressured with nitrogen to 16-20 psia, then heated with agitation to reaction temperature (130°C). The DMC catalyst is activated with 195 g of PO at 130°C under 20 psia nitrogen, and then 810 g PO (1,005 g total) is added continuously (5 g/min) with stirring followed by a 70 minute digest period (130°C) to consume residual oxide. An intermediate sample (105 g) is removed for hydroxyl analysis (4.600% OH or 370 molecular weight corresponding to the four mole propoxylate). To the remaining PO-alcohol adduct 1,320 g of EO is added at (5 g/min) with stirring at 130°C followed by a 60 min digestion period (130°C) with intermediate sampling (453 g). After a second ethylene oxide (445 g) feed and digest period, the reaction product is sampled for hydroxyl analysis: 2.181% hydroxyl or 779 molecular weight, corresponding to the alkoxylate product DIBC/4PO/9EO. As listed in Table 2, the DIBC/4PO/9EO sample contains 0.2 wt% of unreacted alcohol residue and has a PDI at 1.05.

Following the same procedure, other DIBC/PO/EO products are prepared as listed in Table 2. All the samples contain less than 2 wt% unreacted alcohol residue and exhibit a PDI of less than 1.15. The samples show good surface tension reduction capability and low contact angles with the selected samples.

**Table 2. Property Results for DIBC Alkoxylates**

| Description | Unreacted DIBC (wt%) | Surface Tension 1 wt% dynes cm | Contact Angle (deg) | Draves 20 sec. wet time (wt%) | Mw | Pd | Ross Miles Foam 0.1% | | |
|---|---|---|---|---|---|---|---|---|---|
| | | | | | | | Initia l (mm) | 5 Min (mm) | Rate (mm/ min) |
| DIBC/10E O¹ | 1.1 | 42.4 | 96.0 | - | 658 | 1.04 | 75 | 10 | -13 |
| DIBC/4PO/ 7EO | 0.2 | 28.6 | 74.1 | 0.10 | 887 | 1.06 | 0 | 0 | - |
| DIBC/4PO/ 9EO | 0.2 | 26.9 | 75.1 | 0.13 | 991 | 1.05 | 20 | 0 | -4 |
| DIBC/2BO/ 3EO | 0.7 | - | 66.1 | - | 549 | 1.11 | - | - | - |
| DIBC/2BO/ 5EO | 0.4 | 27.1 | 64.2 | 0.10 | 651 | 1.13 | 17 | 0 | -3 |
| DIBC/2BO/ 6EO | 0.3 | 27.9 | 60.9 | 0.12 | 698 | 1.12 | 28 | 0 | -6 |
| DIBC/2BO/ 7EO | 0.3 | 28.0 | 60.2 | 0.10 | 728 | 1.12 | 30 | 0 | -6 |
| DIBC/2BO/ 10EO | 0.2 | 27.7 | 54.1 | 0.12 | 868 | 1.11 | 74 | 7 | -13 |

| | | | | | | | | | |
|---|---|---|---|---|---|---|---|---|---|
| ¹Comparative example | | | | | | | | | |

## Claims

1. A composition comprising one or more alkoxylates of formula II: 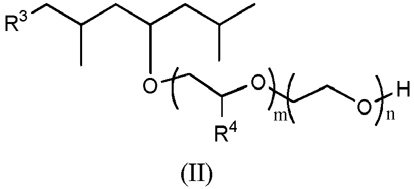 wherein R³ is H or iso-propyl and R⁴ is CH₃ or CH₂CH_{3;} m is 1-6; n is 1-40; wherein the polydispersity index of the alkoxylates is 1.15 or less, and wherein the composition comprises no more than 2 percent by weight of residual alcohol.

2. A process for making the alkoxylate of claim 1, comprising: reacting under alkoxylation conditions a secondary alcohol having 9 to 12 carbon atoms and a branching degree of 3 or more with an alkylene oxide compound containing 3 or more carbon atoms followed by ethylene oxide, wherein the alkoxylation is conducted in the presence of a double metal cyanide catalyst;
wherein the alkylene oxide compound is propylene oxide or butylene oxide; and
wherein the secondary alcohol is 2,6,8-trimethyl-4-nonanol or 2,6-dimethyl heptan-4-ol.

3. A formulation selected from a detergent, hard surface cleaner, polyurethane formulation, epoxy formulation, emulsion polymerization formulation, thermoplastic formulation, metal product, agricultural product including herbicides and pesticides, oilfield product, pulp and paper product, textile formulation, water treatment product, flooring product, ink formulation, colorant formulation, pharmaceutical product, cleaning product, personal care product, fluororesin dispersion, and lubricant, wherein the formulation comprises a composition according to claim 1.

## Patentansprüche

1. Eine Zusammensetzung, die ein oder mehrere Alkoxylate der Formel II beinhaltet: 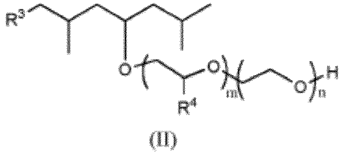 wobei R³ H oder Isopropyl ist und R⁴ CH₃ oder CH₂CH₃ ist; m 1-6 ist; n 1-40 ist; wobei der Polydispersitätsindex der Alkoxylate 1,15 oder weniger ist, und wobei die Zusammensetzung nicht mehr als 2 Gewichtsprozent Restalkohol beinhaltet.

2. Ein Verfahren zum Herstellen des Alkoxylats gemäß Anspruch 1, das Folgendes beinhaltet: unter Alkoxylierungsbedingungen Zur-Reaktion-Bringen eines sekundären Alkohols mit 9 bis 12 Kohlenstoffatomen und einem Verzweigungsgrad von 3 oder mehr mit einer Alkylenoxidverbindung, die 3 oder mehr Kohlenstoffatome enthält, gefolgt von Ethylenoxid, wobei die Alkoxylierung in Gegenwart eines Doppelmetallcyanidkatalysators ausgeführt wird:
wobei die Alkylenoxidverbindung Propylenoxid oder Butylenoxid ist; und
wobei der sekundäre Alkohol 2,6,8-Trimethyl-4-nonanol oder 2,6-Dimethylheptan-4-ol ist.

3. Eine Formulierung, die ausgewählt ist aus einem Detergens, hartem Oberflächenreiniger, Polyurethanformulierung, Epoxidformulierung, Emulsionspolymerisationformulierung, thermoplastischer Formulierung, Metallprodukt, Agrarprodukt einschließlich Herbiziden und Pestiziden, Ölfeldprodukt, Pulpe- und Papierprodukt, Textilformulierung, Wasseraufbereitungsprodukt, Bodenbelagprodukt, Druckfarbenformulierung, Farbmittelformulierung, pharmazeutischem Produkt, Reinigungsprodukt, Körperpflegeprodukt, Fluorharzdispersion und Gleitmittel, wobei die Formulierung eine Zusammensetzung gemäß Anspruch 1 beinhaltet.

## Revendications

1. Une composition comprenant un ou plusieurs alcoxylates de formule II : 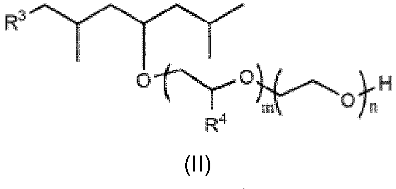 dans laquelle R³ est H ou un iso-propyle et R⁴ est CH₃ ou CH₂CH₃ ; m vaut de 1 à 6 ; n vaut de 1 à 40 ; l'indice de polydispersité des alcoxylates étant de 1,15 ou moins, et la composition ne comprenant pas plus de 2 pour cent en poids d'alcool résiduel.

2. Un procédé pour la fabrication de l'alcoxylate de la revendication 1, comprenant : la mise en réaction dans des conditions d'alcoxylation d'un alcool secondaire ayant de 9 à 12 atomes de carbone et un degré de ramification de 3 ou plus avec un composé d'oxyde d'alkylène contenant 3 atomes de carbone ou plus suivi par de l'oxyde d'éthylène, dans lequel l'alcoxylation est réalisée en présence d'un catalyseur au cyanure métallique double ;
dans lequel le composé d'oxyde d'alkylène est l'oxyde de propylène ou l'oxyde de butylène ; et
dans lequel l'alcool secondaire est le 2,6,8-triméthyl-4-nonanol ou le 2,6-diméthyl heptan-4-ol.

3. Une formulation sélectionnée parmi un détergent, un produit de nettoyage de surface dure, une formulation de polyuréthane, une formulation d'époxy, une formulation de polymérisation en émulsion, une formulation thermoplastique, un produit pour métal, un produit agricole y compris des herbicides et pesticides, un produit de champ de pétrole, un produit pour pâte à papier et papier, une formulation pour textile, un produit de traitement de l'eau, un produit pour revêtement de sol, une formulation pour encre, une formulation pour colorant, un produit pharmaceutique, un produit de nettoyage, un produit d'hygiène personnelle, une dispersion pour fluororésine, et un lubrifiant, la formulation comprenant une composition selon la revendication 1.
